# EUROPEAN PATENT SPECIFICATION

(11) **EP 2 128 147 B1**
(45) Date of publication and mention of the grant of the patent: **15.08.2012**
(21) Application number: 08703880.8
(22) Date of filing: 25.01.2008
(51) Int. Cl.: C07D 301/10, B01J 23/50, B01J 27/232, B01J 37/08, C07D 303/04

(54) **METHOD FOR PRODUCING PROPYLENE OXIDE**
VERFAHREN ZUR HERSTELLUNG VON PROPYLENOXID
PROCÉDÉ DE FABRICATION D'OXYDE DE PROPYLÈNE

(30) Priority: 31.01.2007 JP 2007021174
(43) Date of publication of application: 02.12.2009
(73) Proprietor: Sumitomo Chemical Company, Limited, Tokyo 104-8260 (JP)
(72) Inventor: KANO, Hirotsugu, Ehime, 792-0009 (JP); YAMAMOTO, Michio, Shiga, 520-0246 (JP); YONEMOTO, Tetsuro, Osaka, 560-0003 (JP)
(74) Representative: Vossius & Partner
(86) International application number: PCT/JP2008/051053
(87) International publication number: WO 2008/093605

(56) References cited:
- EP-A1- 1 393 801
- WO-A1-02/051541
- WO-A1-2004/030813
- JP-A- 11 510 817
- JP-A- 2001 526 279
- JP-A- 2006 265 233
- JP-A- 2006 501 066
- US-A- 5 625 084
- US-A- 5 856 534
- US-A1- 2005 027 134

## Description

The present invention relates to a method for producing propylene oxide.

### Background Art

Propylene oxide is industrially important as an intermediate material such as industrial chemical, synthetic resin, or rubber. Methods for producing propylene oxide are known, which comprise reacting propylene and oxygen in the presence of a silver catalyst (See Patent Literature 1 and Patent Literature 2, for example). Further, it is known that propylene oxide can be produced, with the use of a catalyst that contains a small amount of alkali metal, by adding water and a halogen compound to a reaction gas (See Patent Literature 3, for example). However, productivity of propylene oxide by these production methods is not always industrially satisfactory.

### Citation List

Patent Literature 1
   Japanese Patent Application Publication, Tokukaihei, No. 1-231942 A (Publication Date: September 18, 1989)
Patent Literature 2
   Japanese Translation of PCT International Application, Tokuhyou, No. 2002-510306 A (Publication Date: April 2, 2002)
Patent Literature 3
   Japanese Patent Application Publication, Tokukai, No. 2006-265233 A (Publication Date: October 5, 2006)

### Summary of Invention

The present invention provides a method for producing propylene oxide by reacting propylene with oxygen, with efficiency and industrial advantage.

The present invention provides a method for producing propylene oxide which comprises a step of reacting propylene with oxygen in the presence of water, a halogen compound, and a silver catalyst containing alkaline earth metal carbonate as a support, said silver catalyst having 10 µmol/g or more oxygen adsorption capacity as measured by pulse adsorption method, wherein the amount of water used is 0.2 to 10 mols with respect to 1 mole of propylene .

Other objects, features, and superior points of the present invention can be fully understood by the ensuing detailed description. Further, the detailed explanation reveals advantages of the invention.

### Description of Embodiments

The following explains a silver catalyst (hereinafter, referred to as "silver catalyst of the present invention") which contains alkaline earth metal carbonate as a support and which has 10 µmol/g or more oxygen adsorption capacity. The oxygen adsorption capacity of the silver catalyst of the present invention is measured by a pulse adsorption method.

Examples of the alkaline earth metal carbonate used as the support in the present invention include magnesium carbonate, calcium carbonate, strontium carbonate and barium carbonate, preferably calcium carbonate, strontium carbonate and barium carbonate. The specific surface area of the alkaline earth metal carbonate is not specifically limited, but it is preferably 10 to 70 m²/g as measured by a BET method with use of nitrogen adsorption. The amount of the alkaline earth metal carbonate used is typically 0.1 to 1000 times by weight, preferably 0.3 to 200 times by weight, as much as silver in the silver catalyst of the present invention.

The silver catalyst of the present invention is obtained typically by contacting and mixing alkaline earth metal carbonate with metallic silver, a silver compound, or a mixture of them to prepare a silver containing composition, followed by treating the silver containing composition under a reducing condition. The amount of silver in the silver catalyst of the present invention is not specifically limited, and typically used are the catalysts in which the amount of silver is preferably 0.1 or more % by mass, more preferably 0.5 or more % by mass.

Examples of the silver compound include silver oxide, silver carbonate, silver nitrate, silver sulfate, silver cyanide, silver chloride, silver bromide, silver iodide, silver acetate, silver benzonate, silver acetylacetonate, and silver lactate.

An acid, a nitrogen containing compound, or a mixture of them may be added in preparation of the silver containing composition by contacting and mixing alkaline earth metal carbonate with metallic silver, a silver compound, or a mixture of them, when the silver containing composition is produced.

Either organic acid or inorganic acid may be used as the acid. Preferably, an organic acid is used as the acid. Examples of the inorganic acid include hydrochloric acid, nitric acid, nitrous acid, sulfuric acid, and perchloric acid. Examples of the organic acid include aliphatic carboxylic acids such as acetic acid, oxalic acid, propionic acid, butyric acid, citric acid, maleic acid, fumaric acid and tartaric acid, and aromatic carboxylic acids such as benzoic acid, benzenedicarboxylic acid, benzenetricarboxylic acid, naphthalene dicarboxylic acid and anthracene dicarboxylic acid, preferably aliphatic carboxylic acids, in particular preferably oxalic acid and citric acid.

The amount of the acid used is typically 0.1 to 10 mol with respect to one mol of silver in metallic silver, a silver compound, or a mixture of them.

Examples of a nitrogen containing compound include nitrogen containing organic compounds such as an amine compound, an imine compound, an amide compound, an hydrazine compound, a nitrile compound, a nitro compound and a nitroso compound, nitrogen containing inorganic compounds such as ammonia, hydroxylamine, hydrazine and a hydroxylamine hydrochloride, and quaternary ammonium salts, in particular preferably an amine compound. Some of the nitrogen containing compounds form acid addition salts such as amine hydrochloride and amine acetate, these salts of which may be used.

The amount of the nitrogen containing compound used is 0.1 to 20 molar ratios with the silver in the metallic silver and/or the silver compound.

Examples of the amine compound include aliphatic or aromatic amines having the carbon number of 1 to 20, such as methylamine, ethylamine, propylamine, butylamine, amylamine, hexylamine, heptylamine, octylamine, decylamine, dodecylamine, stearylamine, dimethylamine, diethylamine, dipropylamine, dibutylamine, trimethylamine, triethylamine, ethanolamine, dimethylethanolamine, diethanolamine, triethanolamine, ethylenediamine, tetramethylenediamine, pentamethylenediamine, diethylenetriamine, aniline, benzylamine and phenylenediamine, and amino acids such as glycine.

Examples of the imine compound include ethyleneimine, pyrrolidine, piperidine and piperazine.

Examples of the amide compound include acetamide and benzamide.

Examples of the hydrazine compound include hydrazine, methylhydrazine, and phenylhydrazine.

Examples of the nitrile compound include benzonitrile, and butyronitrile.

Examples of the nitro compound include nitrobenzene, and nitropyridine.

Examples of the nitroso compound include nitrosodimethylaniline, and nitrosonaphthol.

Examples of the quaternary ammonium salts include quaternary ammonium hydroxides such as tetramethylammonium hydroxide, tetraethylammonium hydroxide and tetrapropylammonium hydroxide, and quaternary ammonium halides such as tetramethylammonium chloride, tetramethylammonium bromide, tetraethylammonium chloride and tetraethylammonium bromide.

The silver containing composition is prepared, for example, by contacting and mixing the silver compound and the alkaline earth metal carbonate at a temperature of 0 to 200 °C in a solvent such as water, methanol, ethanol, propanol, tetrahydrofuran, toluene, or hexane, and then subjecting thus obtained mixture to condensation. If necessary, an acid, a nitrogen containing compound, or a mixture of them may be added to prepare the silver containing composition.

The silver catalyst of the present invention is prepared by reducing the silver containing composition in flow of reducing gas such as hydrogen, carbon monoxide, methane, ethane, propane, butane, ethylene, propylene, butene and butadiene, or mixture of them. The reducing gas may be used after being mixed at an appropriate ratio with a diluting gas such as nitrogen, helium, argon, and water vapor. An optimal temperature for the reduction varies depending on the kind and the composition of the gas, however, too high temperature may cause agglomeration of silver particles, resulting in smaller metallic surface area of silver. Accordingly, the temperature for the reduction is typically 20 to 300 °C, the upper limit of which is preferably 250 °C, and more preferably 220 °C. The silver containing composition may be reduced after molding it, or the silver catalyst obtained by the reduction may be molded.

The adsorption amount of oxygen molecules with respect to the silver catalyst of the present invention is measured by pulse adsorption method. The measurement of the adsorption amount of oxygen molecules by the pulse adsorption method is carried out based on the method described in Journal of Catalysis, Vol. 139, pp. 41 - 47, 1993. The measurement may be carried out by using a fully automatic temperature-programmed desorption spectrometer apparatus TPD-1-ATw (manufactured by Bel Japan Inc.) or an apparatus model having performance equivalent to the apparatus.

Next, the following explains a method for producing propylene oxide by reacting propylene with oxygen, in the presence of the silver catalyst of the present invention, water, and a halogen compound (hereinafter, this method will be referred to as "the reaction of the present invention").

The silver catalyst of the present invention may be used in any amount not less than its catalytic effective amount, the used amount of which is typically 0.00005 or more mol in metallic silver equivalent with respect to 1 mol of propylene. Its upper limit of the amount of the silver catalyst is not specifically limited, which may be determined as appropriate in consideration of economical efficiency.

Water may be mixed with the halogen compound, oxygen and propylene gas, and then provided as a mixed gas in the reaction. The water contained in the mixed gas may be water vapor. The amount of water used is typically 0.1 to 20 mol, 0.2 to 10 mol, and preferably 0.3 to 8 mol, with respect to 1 mol of propylene.

The halogen compound is preferably a saturated or unsaturated organic halogen compound such as an organic fluoride, an organic chloride, an organic bromide and an organic iodide, and it is most preferably an organic chloride, more specifically ethyl chloride, 1,2-ethylene dichloride, methyl chloride, or vinyl chloride. The halogen compound preferably exists as gas under temperature and pressure conditions in a reaction system. Regarding the used amount of the supplied halogen compound, its optimal level varies depending on factors such as the concentration of olefin, the concentration of oxygen, the amount of catalyst, the amount of water used, which level is typically 1 to 1000 ppm and more preferably 1 to 500 ppm in a mixed gas except water.

As the oxygen, oxygen alone may be used, and a mixed gas containing gas inactive to the reaction, such as the air, may be used. The amount of oxygen used varies depending on the reaction type, the kind of catalyst, reaction temperature or the like. The amount of oxygen is typically 0.01 to 100 mol, and preferably 0.03 to 30 mol, with respect to 1 mol of propylene. The reaction temperature is typically 100 to 400 °C, and preferably 120 to 300 °C.

The present reaction is carried out under the reaction pressure in the range of the reduced pressure to the increased pressure. By carrying out the reaction in coexistence of water and the halogen compound under such a reaction pressure condition, productivity and selectivity of propylene oxide can be improved. The reduced pressure means a condition in which the reaction pressure is reduced to be lower than an atmospheric pressure. The increased pressure means a condition in which the reaction pressure is increased to be higher than the atmospheric pressure. Under the condition of the reduced pressure to the increased pressure, the pressure is typically in the range of 0.01 to 3 MPa, and preferably in the range of 0.02 to 2 MPa, in the absolute pressure.

The reaction of the present invention may be carried out as batch reaction or continuous reaction, preferably as continuous reaction for industrial application. The reaction of the present invention may be carried out by mixing and contacting the silver catalyst, water, propylene, oxygen and the halogen compound under the condition in which the reaction pressure is set at the reduced pressure to the increased pressure. After completing the reaction, target propylene oxide can be separated by collecting reaction solution or reaction gas, followed by subjecting it to carry out general separation means such as distillation.

### [Examples]

The following explains the present invention further in detail by Examples. However, the present invention is not limited to these Examples.

### Measurement Method for Adsorption Amount of Oxygen Molecules in Silver Catalyst By Adsorption Method

A typical procedure of the pulse adsorption method in the present invention is explained below.

As a measurement device, a fully automatic temperature-programmed desorption spectrometer apparatus TPD-1-ATw (manufactured by Bel Japan Inc.) was used. Organic substances on the surface of a catalyst were calcined and removed by oxygen gas flowing through 0.5g of the catalyst at 50 ml/min, at 200 °C, for 60 minutes; the catalyst was reduced with hydrogen gas flowing at 50 ml/min, at 200 °C, for 60 minutes, followed by purging with helium flowing at 50 ml/min for 15 minutes, and then 0.98 ml of oxygen was pulse-injected at 200 °C several times with helium flowing at 50ml/min to absorb oxygen molecules on the catalyst, followed by measuring adsorption amount.

### Reference Example 1

At 20 to 25 °C, 100 g of a silver nitrate solution containing 10 g of silver nitride was dropped into 245.0 g of a slurry containing, as alkaline earth metal carbonate, 23.5 g of strontium carbonate (produced by Sakai Chemical Industry Co., Product Name: SW-K20) having the surface area of 19.0 m²/g, and kept being stirred for three hours. Solids were filtrated and washed with 200mL of ion exchanged water three times to give a mixture of silver carbonate and strontium carbonate.

### Example 1

Filling a glass tube for calcination with the mixture of silver carbonate and strontium carbonate obtained in Reference Example 1, the mixture was subjected to reduction at 110 °C for one hour with a mixed gas of CO (10 %) and N₂ (90 %) flowing at 100mL/min. Subsequently, the temperature was raised to 210 °C over 5 hours, and then, the mixture was calcined for 1 hour to give a silver catalyst. Next, the adsorption amount of oxygen molecules was measured by the pulse adsorption method. Taking out 0.5 g of the catalyst, the adsorption amount of oxygen molecules was measured under the above-mentioned condition. The adsorption amount of oxygen molecules in thus obtained silver catalyst according to the pulse adsorption method was 24.2 µmol/g.

Filling a 1/2-inch reaction tube made of stainless steel with 1mL of thus obtained silver catalyst, the reaction tube was supplied with 450 mL/Hr of propylene, 900 mL/Hr of air, 990 mL/Hr of nitrogen gas, 1.2 mL/Hr of water, and 50 ppm of ethyl chloride to carry out the reaction at the reaction temperature of 200 °C under the condition of the increased pressure (equivalent to 0.4 MPa in the absolute pressure). Propylene conversion was 9.9 % and propylene oxide yield was 460 µmol/Hr.

### Comparative Example 1

A silver catalyst was obtained by reduction and calcination in the same manner as Example 1 except that the mixture of silver carbonate and strontium carbonate obtained in Reference Example 1 was calcined at 350 °C for three hours in air flow of 100 mL/min. The adsorption amount of oxygen molecules in thus obtained silver catalyst was measured by the pulse adsorption method. The adsorption amount of oxygen molecules was 6.5 µmol/g.

Except that 1 mL of this silver catalyst was used, the same reaction as Example 1 was carried out. Propylene conversion was 2.3 % and propylene oxide yield was 210 µmol/Hr.

### Reference Example 2

A silver containing composition was obtained by carrying out of the same preparation as Reference Example 1 except that 4.37 g of sodium hydroxide was added to the slurry solution of strontium carbonate.

### Example 2

The silver containing composition obtained in Reference Example 2 was reduced and calcined under the same conditions as Example 1 to give a silver catalyst. The adsorption amount of oxygen molecules in thus obtained silver catalyst was measured by the pulse adsorption method, and the adsorption amount of oxygen molecules was 51.4 µmol/g. As a result of carrying out a reaction in the same manner as Example 1 except that 1 mL of this silver catalyst was used, propylene conversion was 6.6 % and propylene oxide yield was 390 µmol/Hr.

### Example 3

Three (3) g of the silver catalyst obtained in Example 2 was dispersed in 10 g of water, and then 28.3 mg of sodium acetate was added, followed by removing water with an evaporator to give an Na added silver catalyst. As a result of carrying out the same reaction as Example 1 except that the obtained silver catalyst was used, propylene conversion was 9.4 % and propylene oxide yield was 320 µmol/Hr.

### Reference Example 3

A silver containing composition was prepared by replacing silver nitrate with silver sulfate in Reference Example 1. This composition was reduced and calcined in the same procedure as Example 1 except that a mixed gas of propylene (10 %) and N₂ (90 %) was used as reducing gas to give a silver catalyst. The adsorption amount of oxygen molecules in the obtained silver catalyst was measured by the pulse adsorption method. The adsorption amount of oxygen molecules was 17.4 µm/g.

### Example 4

A reaction was carried out in the same manner as Example 1 except that 1 mL of the silver catalyst obtained in Reference Example 3 was used. In this reaction, propylene conversion was 8.4 % and propylene oxide yield was 370 µmol/Hr.

The present invention makes it possible to produce propylene oxide industrially advantageously.

The embodiments and concrete examples discussed in the foregoing detailed explanation serve solely to illustrate the technical details of the present invention, which should not be narrowly interpreted within the limits of such embodiments and concrete examples, but rather may be applied in many variations within the spirit of the present invention, provided such variations do not exceed the scope of the patent claims set forth below.

## Claims

1. A method for producing propylene oxide which comprises a step of reacting propylene with oxygen in presence of water, a halogen compound, and a silver catalyst containing alkaline earth metal carbonate as a support, said silver catalyst having 10 µmol/g or more oxygen adsorption capacity as measured by pulse adsorption method, wherein the amount of water used is 0.2 to 10 mols with respect to 1 mol of propylene.

2. The method for producing propylene oxide as set forth in claim 1, wherein the halogen compound is an organic halogen compound and the amount of the halogen compound used is 1 to 1000 ppm with respect to the total amount of a mixed gas except water.

3. The method for producing propylene oxide as set forth in claim 1, wherein the amount of silver in the silver catalyst is 0.5 or more % by mass.

4. The method for producing propylene oxide as set forth in claim 1, wherein the silver catalyst is a silver catalyst obtained by reducing a silver containing composition, said silver containing composition being obtained by contacting metallic silver with alkaline earth metal carbonate.

## Patentansprüche

1. Ein Verfahren zur Herstellung von Propylenoxid, das einen Schritt des Umsetzens von Propylen mit Sauerstoff in Gegenwart von Wasser, einer Halogenverbindung und eines Silberkatalysators, enthaltend Erdalkalimetallcarbonat als einen Träger, umfasst, wobei der Silberkatalysator ein durch ein Puls-Adsorptionsverfahren gemessenes Sauerstoff-Adsorptionsvermögen von 10 µMol/g oder mehr aufweist und die eingesetzte Wassermenge 0,2 bis 10 Mol, bezogen auf 1 Mol Propylen, beträgt.

2. Das Verfahren zur Herstellung von Propylenoxid wie in Anspruch 1 beschrieben, wobei die Halogenverbindung eine organische Halogenverbindung ist und die eingesetzte Menge der Halogenverbindung 1 bis 1000 ppm, bezogen auf die Gesamtmenge eines Gasgemisches mit Ausnahme von Wasser, beträgt.

3. Das Verfahren zur Herstellung von Propylenoxid wie in Anspruch 1 beschrieben, wobei die Silbermenge im Silberkatalysator 0,5 oder mehr Massen% beträgt.

4. Das Verfahren zur Herstellung von Propylenoxid wie in Anspruch 1 beschrieben, wobei der Silberkatalysator ein durch Reduktion einer silberhaltigen Zusammensetzung erhaltener Silberkatalysator ist, wobei die silberhaltige Zusammensetzung durch in Kontakt bringen von metallischem Silber mit Erdalkalimetallcarbonat erhalten wird.

## Revendications

1. Procédé pour produire de l'oxyde de propylène qui comprend une étape de réaction du propylène avec l'oxygène en présence d'eau, d'un composé halogéné et d'un catalyseur à l'argent contenant un carbonate de métal alcalino-terreux comme support, ledit catalyseur à l'argent ayant une capacité d'adsorption de l'oxygène de 10 µmol/g ou plus, mesurée par un procédé d'adsorption à impulsions, où la quantité d'eau utilisée est 0,2 à 10 moles par mole de propylène.

2. Procédé pour produire de l'oxyde de propylène selon la revendication 1 où le composé halogéné est un composé halogéné organique et la quantité du composé halogéné utilisée est 1 à 1000 ppm par rapport à la quantité totale d'un gaz mixte sauf l'eau.

3. Procédé pour produire de l'oxyde de propylène selon la revendication 1 où la quantité d'argent dans le catalyseur à l'argent est 0,5 ou plus % en masse.

4. Procédé pour produire de l'oxyde de propylène selon la revendication 1 où le catalyseur à l'argent est un catalyseur à l'argent obtenu par réduction d'une composition contenant de l'argent, ladite composition contenant de l'argent étant obtenue par mise en contact d'argent métallique avec un carbonate de métal alcalino-terreux.
